# EUROPEAN PATENT APPLICATION

(11) **EP 2 929 905 A1**
(43) Date of publication of application: **14.10.2015**
(21) Application number: 13859736.4
(22) Date of filing: 05.12.2013
(51) Int. Cl.: A61M 15/00

(54) **MEDICINAL INHALATION DEVICE**

(30) Priority: 07.12.2012 JP 2012268602
(71) Applicant: Nippharma Co., Ltd., Tokyo 103-0027 (JP)
(72) Inventor: ONO, Shinichi, Tokyo 103-0027 (JP)
(74) Representative: Moser Götze & Partner Patentanwälte mbB
(86) International application number: PCT/JP2013/082666
(87) International publication number: WO 2014/088055

(57) **Abstract**

[Problem] To provide an inhalation device with a simple structure which is capable of easily separating a base sheet and a lid sheet.

[Solution] The present invention is an inhalation device (20) which is capable of tearing off a lid sheet (12) from a base sheet (11) of a medicine holding sheet (10). The inhalation device (20) comprises a base spool (30) on which the base sheet (11) is wound, a lid spool (40) on which the lid sheet (12) is wound and an air piece (50) which is provided at a position opposing a blister (11a) of the base sheet (11) from which the lid sheet (12) has been separated and which has an inlet through which medicine passes. In the present invention, the air piece (50) has a wedge portion (51) which is inserted between the base sheet (11) and the lid sheet (12) at a separation point (60) where the lid sheet (12) separates from the base sheet (11).

## Description

### Technical Field

The present invention relates to a medicinal inhalation device. Specifically, the medicinal inhalation device according to the present invention is adapted for oral administration of a powdered medicine to a patient, for example, and the patient can inhale the medicine via the present device.

### Background Art

Conventionally are known devices adapted to let a patient inhale a medicine suppressing airway inflammation or accelerating bronchial dilatation, for example (Patent Literature 1, Patent Literature 2, and the like). Since each of such devices is adapted to directly administer a powdered medicine to an airway or a lung which is an affected area of inflammation with use of the patient's own sucking force, even a small amount of medicine can be applied to the affected area in a focused manner, and the device has advantages of enabling to suppress generation of a side effect and quickly exerting an effect.

As illustrated in Patent Literature 1 and Patent Literature 2, a general inhalation device has a strip-like medicinal carrier encapsulating a medicine housed therein in a rolled state. The strip-like medicinal carrier is formed by attaching inside surfaces of elongated base sheet and lid sheet to each other, and this base sheet is provided with a plurality of blisters each forming a space to encapsulate the medicine to be spaced from each other in a longitudinal direction thereof. Each blister of the base sheet encapsulates the desired medicine as much as appropriate for a dose, and at the time of administration, the lid sheet is peeled from the base sheet to expose an appropriate amount of medicine.

The general inhalation device also includes a spool adapted to roll up the base sheet and a spool adapted to roll up the lid sheet and rotates these spools in association with each other to give tensile forces to the base sheet and the lid sheet. When the tensile forces are applied to the base sheet and the lid sheet in mutually opposite directions, these sheets are peeled at a separation point, and the medicine encapsulated in the blister is exposed. The inhalation device is provided in the vicinity of the separation point of the sheets with an opening for medicinal inhalation. The patient can inhale the medicine through the opening of the device.

### Citation List

### Patent Literature

Patent Literature 1: JP 2004-512147 W
Patent Literature 2: JP 2005-506164 W

### Summary of Invention

### Technical Problem

However, in the conventional inhalation device, the base sheet and the lid sheet are separated only by rotating forces of the spool adapted to roll up the base sheet and the spool adapted to roll up the lid sheet. Hence, when the lid sheet is peeled from the base sheet, a relatively strong force is required, and a mechanism for generating the force is complicated. For example, in the conventional inhalation device, since both the base sheet and the lid sheet are pulled to generate a force to separate the sheets, at least a mechanism for rotating both the spool adapted to roll up the base sheet and the spool adapted to roll up the lid sheet is required. Such a complicated structure of the inhalation device causes not only a problem of an increase in manufacturing cost but also a problem of high potentiality for a device failure.

For this reason, there is currently a demand for an inhalation device having a simple structure that can separate the base sheet and the lid sheet easily even with a weak force.

### Solution to Problem

As the result of concerted study directed towards solving the aforementioned conventional problem, the present inventors have discovered that providing an air piece having an inhalation opening for a medicine with a wedge portion to be inserted between a base sheet and a lid sheet enables the base sheet and the lid sheet to be separated easily even with a weak force. The present inventors have arrived at the present invention that, since forming the wedge portion can help separation of the lid sheet from the base sheet, only a mechanism of rotating a spool for rolling up the lid sheet has to be provided, for example, and a structure of an inhalation device can further be simplified.

More specifically, the present invention is configured as below.

The present invention relates to an inhalation device 20.

The inhalation device 20 according to the present invention has housed therein a medicinal holding sheet 10. This medicinal holding sheet 10 includes an elongated base sheet 11 having a plurality of blisters 11a encapsulating a medicine and an elongated lid sheet 12 attached to the base sheet 11. The inhalation device 20 can peel the lid sheet 12 from the base sheet 11.

The inhalation device 20 includes a base spool 30, a lid spool 40, and an air piece 50.

The base sheet 11 is wound around the base spool 30. The lid sheet 12 is wound around the lid spool 40. Both the base spool 30 and the lid spool 40 do not necessarily have to be driven to be rotated, and only the lid spool 40 may have a rotary driving mechanism, for example.

The air piece 50 is provided at a position opposed to the blister 11a of the base sheet 11 from which the lid sheet 12 is separated and has an inhalation opening through which the medicine passes.

In the present invention, the air piece 50 has a wedge portion 51 to be inserted between the base sheet 11 and the lid sheet 12 at a separation point 60 at which the lid sheet 12 is separated from the base sheet 11. Meanwhile, the separation point 60 means a position at which the lid sheet 12 is separated from the base sheet 11.

Also, the wedge portion 51 extends from a main body of the air piece 50 to a position at which a tip end thereof reaches the separation point 60.

As in the above configuration, by providing the air piece 50 with the wedge portion 51 and inserting this wedge portion 51 between the base sheet 11 and the lid sheet 12, the sheets are separated easily. Also, by providing the air piece 50 with the wedge portion 51, the base sheet 11 and the lid sheet 12 are separated in the vicinity of the air piece 50 having the inhalation opening for the medicine, and thus the medicine encapsulated in the blister 11a of the base sheet 11 can be prevented from dropping. Further, according to the present invention, since the base sheet 11 and the lid sheet 12 are separated easily even with a weak force, both the base spool 30 and the lid spool 40 do not necessarily have to be driven to be rotated, and separation of the sheets can be achieved by providing only the lid spool 40 with a rotary driving mechanism, for example. Accordingly, the structure of the inhalation device can be simplified.

The inhalation device according to the present invention preferably further includes an indexing wheel 70 which rotates in a state in which the medicinal holding sheet 10 abuts on a circumferential side surface of the indexing wheel. In this case, the wedge portion 51 of the air piece 50 is preferably formed at a position at which the separation point 60 is formed on the circumferential side surface of the indexing wheel 70.

As in the above configuration, when the medicinal holding sheet 10 is separated into the base sheet 11 and the lid sheet 12, by interposing the base sheet between the tip end of the wedge portion 51 and the circumferential side surface of the indexing wheel 70, for example, the base sheet 11 and the lid sheet 12 are separated more easily.

The inhalation device according to the present invention preferably further includes a guide portion 80 on which an outer surface of the lid sheet 12 abuts when the lid sheet 12 is peeled from the base sheet 11 at the separation point 60 and advances in an opposite direction of the base sheet 12. In this case, the tip end of the wedge portion 51 is preferably located between the indexing wheel 70 and the guide portion 80.

As in the above configuration, the indexing wheel 70 and the guide portion 80 are arranged at positions opposed to each other with the medicinal holding sheet 10 interposed therebetween. At this time, an outer surface of the base sheet 12 abuts on the circumferential side surface of the indexing wheel 70 while the outer surface of the lid sheet 12 abuts on the guide portion 80. By locating the tip end of the wedge portion 51 in a space generated between the indexing wheel 70 and the guide portion 80, the base sheet 11 and the lid sheet 12 are separated more easily.

In the present invention, an angle of the mutually separated base sheet 11 and lid sheet 12 with the separation point 60 serving as a vertex is preferably 1 or more and less than 90 degrees.

As in the above configuration, the angle of the base sheet 11 and the lid sheet 12 with the separation point 60 serving as the vertex is less than 90 degrees to cause the sheets to be separated easily even when a force to be applied to the sheets is weak. That is, in a case in which the angle with the separation point 60 serving as the vertex is 90 or more degrees in separating the lid sheet 12 from the base sheet 11, both the base sheet 11 and the lid sheet 12 need to be pulled and rolled up, in which case relatively large tensile forces must be applied to both the sheets. Conversely, by employing the above configuration in which the tip end of the wedge portion 51 is located between the indexing wheel 70 and the guide portion 80, the angle of the base sheet 11 and lid sheet 12 with the separation point 60 serving as the vertex can be less than 90 degrees, and this enables both the sheets to be separated easily.

In the present invention, at least the wedge portion 51 out of the air piece 50 is preferably made of a flexible material.

As in the above configuration, the wedge portion 51 is made of the flexible material to cause the wedge portion 51 to be inserted between the base sheet 11 and the lid sheet 12 effectively.

In the present invention, at least the wedge portion 51 out of the air piece 50 preferably contains an antistatic material.

As in the above configuration, the wedge portion 51 contains the antistatic material to enable to prevent the powdered medicine from attaching to the wedge portion 51 due to the effect of static electricity. Accordingly, it is possible to let a patient inhale an appropriate dose of medicine in an appropriate manner and to prevent leakage of the powdered medicine.

The inhalation device according to the present invention preferably further includes a holding sheet housing chamber 90 in which the medicinal holding sheet 10 is housed in a rolled state and a base sheet housing chamber 100 in which the base sheet 11 wound around the base spool 30, centering on the base spool 30, is housed. In this case, no partition wall for partitioning a space into the respective housing chambers preferably exists between the holding sheet housing chamber 90 and the base sheet housing chamber 100. In a state in which all of the blisters 11a are unopened, the rolled medicinal holding sheet 10 is preferably partially located in the base sheet housing chamber 100. In a state in which all of the blisters 11a are opened, the base sheet 11 wound around the base spool 30 is preferably partially located in the holding sheet housing chamber 90.

As in the above configuration, by omitting a partition wall between the holding sheet housing chamber 90 and the base sheet housing chamber 100, a distance between the holding sheet housing chamber 90 and the base sheet housing chamber 100 can be shortened. Accordingly, the structure of the inhalation device can be compact.

In the inhalation device according to the present invention, the air piece 50 is preferably replaceable.

As in the above configuration, since the air piece 50 having the inhalation opening is replaceable, the air pieces having different inhalation dispersion characteristics can be selectively used for respective patients. For example, by preparing multiple kinds of air piece such as an air piece having a screw-shaped inhalation opening and air pieces having mesh structures with different aperture dimensions, the dispersion characteristics of the powdered medicine can be controlled in accordance with the patient.

### Advantageous Effects of Invention

The present invention can provide an inhalation device having a simple structure that can separate a base sheet and a lid sheet easily even with a weak force.

### Brief Description of Drawings

Fig. 1 illustrates an overview of a medicinal holding sheet housed in an inhalation device.
Fig. 2 illustrates an overview of an internal structure of the inhalation device.
Fig. 3 illustrates the overview of the internal structure of the inhalation device.
Fig. 4 is an enlarged view of a structure around an air piece and an indexing wheel.
Fig. 5 illustrates comparison between a state in which the medicinal holding sheet in an unused state is located in a holding sheet housing chamber and a state in which a base sheet in a used state is located in a base sheet housing chamber.
Figs. 6(a) to 6(c) are perspective views illustrating an example of appearance of an inhalation device.
Figs. 7(a) to 7(c) are perspective views illustrating an example of a gear mechanism of the inhalation device.
Figs. 8(a) to 8(d) are perspective views illustrating an example of a lock mechanism of the inhalation device.
Fig. 9 is a perspective view illustrating an example of an association mechanism of a counter dial in the inhalation device.

### Description of Embodiments

Hereinbelow, embodiments of the present invention will be described with reference to the drawings. The present invention is not limited to the embodiments described below and includes appropriate modifications of the following embodiments within the scope apparent to those skilled in the art.

It is to be noted that, in the present description, "A to B" means "A or more and B or less."

### (1. Medicinal Holding Sheet)

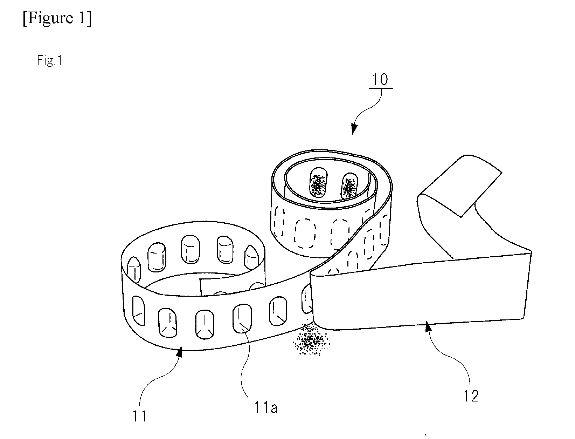

Fig. 1 illustrates an overview of a medicinal holding sheet 10 housed in an inhalation device according to the present invention. As illustrated in Fig. 1, the medicinal holding sheet 10 is elongated and formed in a strip shape. The medicinal holding sheet 10 is formed at least by attaching inside surfaces of a base sheet 11 and a lid sheet 12 to each other. The base sheet 11 has a plurality of blisters 11a which are raised to encapsulate medicines. The blisters 11a are provided at regular intervals along a longitudinal direction of the base sheet 11. Each blister 11a is preferably formed to extend in a direction (shorter direction) perpendicular to the longitudinal direction of the base sheet 11. This blister 11a has encapsulated therein an appropriate dose of medicine for single inhalation. Meanwhile, the base sheet 11 may have at a tip end thereof an idle area provided with no blisters 11a. Also, the lid sheet 12 is attached to an area of the base sheet 11 with no blisters 11a so as to be separable from each other. By doing so, the blisters 11a of the base sheet 11 are sealed by the lid sheet 12. Meanwhile, in a case in which the base sheet 11 has the idle area provided with no blisters 11 a, the lid sheet 12 does not need to be attached to this idle area. Each of the base sheet 11 and the lid sheet 12 is preferably made of a plastic material, an aluminum material, or a laminated body thereof. As an attaching method of the base sheet 11 and the lid sheet 12, heat-sealed adhesion can suitably be employed.

It is preferable to encapsulate a powdered medicine in the blister 11a of the medicinal holding sheet 10. However, a liquid medicine, a solid medicine, or the like can be encapsulated in the blister 11a. The inhalation device according to the present invention can suitably be used for oral administration to a patient of a medicine especially for treatment of respiratory diseases such as asthma, a chronic obstructive pulmonary disease (COPD), bronchitis, and a chest infection. As for the medicine, known one may be used. For example, as the medicine to be encapsulated in the medicinal holding sheet 10, one or more kinds can be selected from among analgesics such as codeine, dihydromorphine, ergotamine, fentanyl, and morphine; anti-anginal medicines such as diltiazem, anti-allergic medicines such as a cromoglicic acid compound, ketotifen, and nedocromil; anti-infective medicines such as cephalosporins and penicillins; antihistamine medicines such as methapyrilene; anti-inflammatory agents such as beclomethasone, fluticasone, and flunisolide; antitussive medicines such as noscapine; and bronchodilators such as albuterol, salmeterol, ephedrine, adrenaline, and fenoterol.

### (2. Internal Structure of Inhalation Device)

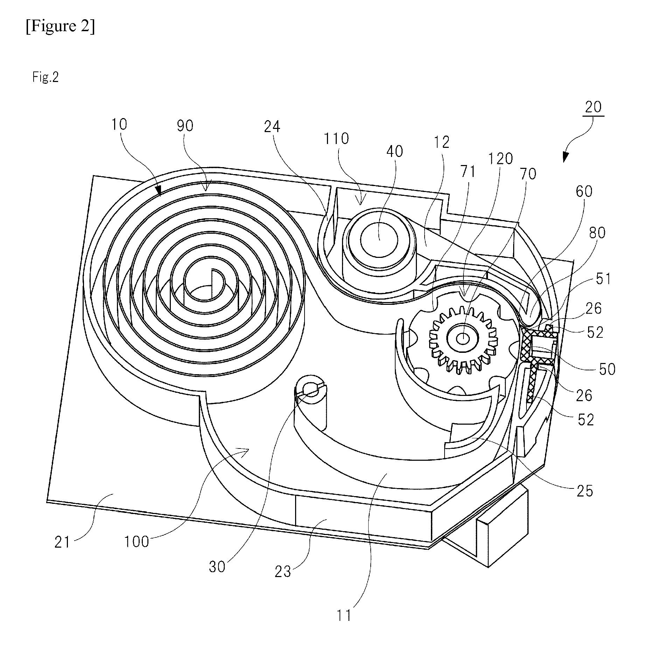

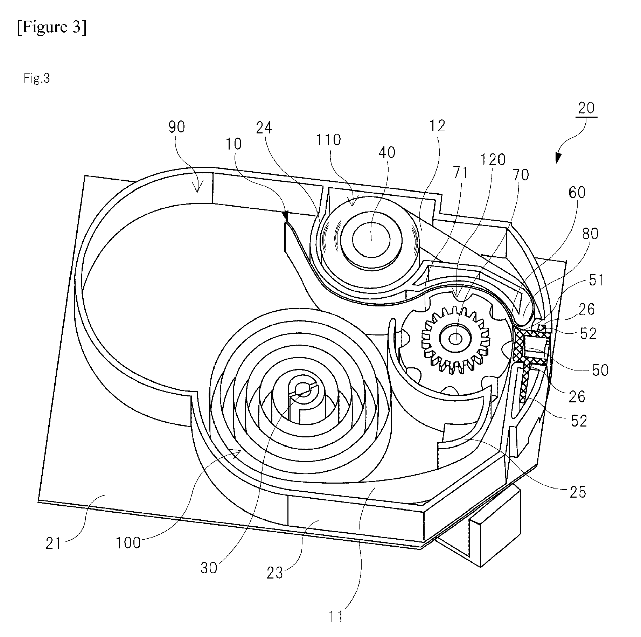

Figs. 2 and 3 illustrate an overview of an internal structure of an inhalation device 20. As illustrated in Figs. 2 and 3, the aforementioned medicinal holding sheet 10 is housed in the inhalation device 20. Fig. 2 illustrates a state in which the medicinal holding sheet 10 is unused while Fig. 3 illustrates a state after the medicinal holding sheet 10 has started to be used. Meanwhile, in Figs. 2 and 3, illustration of the blisters 11a of the base sheet 11 illustrated in Fig. 1 is omitted.

As illustrated in Figs. 2 and 3, the inhalation device 20 includes an external wall consisting of a bottom plate 21, a ceiling plate (illustration thereof is omitted) facing the bottom plate 21, and a side plate 23 connecting the bottom plate 21 with the ceiling plate. Meanwhile, in Figs. 2 and 3, the inhalation device 20 is drawn with illustration of the ceiling plate omitted to show the internal structure of the inhalation device 20. The external wall of the inhalation device 20 defines an internal space in which the medicinal holding sheet 10 can be housed. The external wall of the inhalation device 20 may be provided with a hole adapted to exhaust the medicine that the patient cannot inhale up to prevent clogging caused by the medicine. For example, the exhaust hole for the medicine is provided on an opposite side of an inhalation opening for the medicine.

As illustrated in Figs. 2 and 3, in the present embodiment, the internal space of the inhalation device 20 is partitioned into a holding sheet housing chamber 90, a base sheet housing chamber 100, a lid sheet housing chamber 110, and an indexing wheel housing chamber 120. The holding sheet housing chamber 90, the base sheet housing chamber 100, the lid sheet 110, and the indexing wheel housing chamber 120 are provided adjacent to each other. More specifically, in the present embodiment, the lid sheet housing chamber 110 is defined by being surrounded by the external wall and a first internal wall 24 provided to extend from this external wall to partition the internal space except a passage of the lid sheet 12. Also, the indexing wheel housing chamber 120 is defined by being surrounded by the external wall, the first internal wall 24, and a second internal wall 25 partitioning the internal space without being connected with the external wall except an entrance of the medicinal holding sheet 10 and an exit of the base sheet 11. On the other hand, the internal space except the aforementioned lid sheet housing chamber 110 and indexing wheel housing chamber 120 is the holding sheet housing chamber 90 and the base sheet housing chamber 100 except a passage of the sheets. That is, no internal wall for partitioning the space exists between the holding sheet housing chamber 90 and the base sheet housing chamber 100. Thus, the rolled medicinal holding sheet 10 is housed in the holding sheet housing chamber 90 for the most part, but in a state in which all of the blisters 11 a are unopened, the medicinal holding sheet 10 is partially located in the base sheet housing chamber 100 as well. Also, the base sheet 11 wound around a base spool 30 is housed in the base sheet housing chamber 100 for the most part, but in a state in which all of the blisters 11 a are opened, the base sheet 11 is partially located in the holding sheet housing chamber 90 as well. In this manner, by providing no internal wall between the holding sheet housing chamber 90 and the base sheet housing chamber 100 to shorten a distance therebetween, the entire device is configured to be compact. This will be described below in detail.

As illustrated in Fig. 2, the medicinal holding sheet 10 is first housed in the holding sheet housing space 90. That is, in the holding sheet housing chamber 90, the medicinal holding sheet 10 before being separated into the base sheet 11 and the lid sheet 12 is housed in a rolled state. It is to be noted that, although illustration of the blisters 11a of the base sheet 11 is omitted in Fig. 2, the medicinal holding sheet 10 is rolled as firmly as to prevent these blisters 11a from crushing under pressure and is housed in the holding sheet housing chamber 90. The tip end of the medicinal holding sheet 10 is pulled out and is guided to the indexing wheel housing chamber 120. The medicinal holding sheet 10 is separated into the base sheet 11 and the lid sheet 12 at the indexing wheel housing chamber 120. The indexing wheel housing chamber 120 is provided with a rotatable indexing wheel 70, and the separated base sheet 11 advances along with rotation of the indexing wheel 70 and is guided to the base sheet housing chamber 100. On the other hand, the lid sheet housing chamber 110 is provided with a lid spool 40 for rolling up the lid sheet 12, and the separated lid sheet 12 is rolled up in the lid spool 40 to advance in an opposite direction of the base sheet 11 and is guided to the lid sheet housing chamber 110.

As illustrated in Figs. 2 and 3, the base sheet housing chamber 100 is provided with the base spool 30. The base spool 30 is erected on the bottom plate 21 or the ceiling plate and stands vertically to a plane of the bottom plate 21 or the ceiling plate. In the present embodiment, the base spool 30 is formed approximately in a cylindrical shape and is provided at a center thereof with a slit from an upper portion to a lower portion thereof. In this slit, a tip end of the base sheet 11 can be inserted and stop in an engaged state. In this manner, the base sheet 11 is wound around the base spool 30. The base spool 30 may be coupled with a base spool gear (described below) for rotary drive. Hence, the base spool 30 is rotated in a circumferential direction, centering on a rotation shaft extending in a direction perpendicular to the bottom plate 21. As a rotary driving mechanism for rotating the base spool 30, a known one can be employed. For example, as the rotary driving mechanism, a ratchet mechanism, in which the lid spool 40 is rotated in one direction but is restricted to be rotated in an opposite direction thereof, may be employed. Also, the base spool gear coupled with the base spool 30 is in association with a center gear (described below), for example, so that the base spool 30 may be rotated as much as a predetermined amount along with turning of the center gear. However, in the present embodiment, the base spool 30 does not need to be rotated, and the rotary driving mechanism for the base spool 30 can be omitted.

Also, as illustrated in Figs. 2 and 3, the lid sheet housing chamber 110 is provided with the lid spool 40. The lid spool 40 is erected on the bottom plate 21 or the ceiling plate and stands vertically to the plane of the bottom plate 21 or the ceiling plate in a similar manner to the aforementioned base spool 30. In the present embodiment, the lid spool 40 is formed approximately in a cylindrical shape, and a tip end of the lid sheet 12 can be attached to a circumferential surface of the lid spool 40 by adhesive or the like. Alternatively, the lid spool 40 may be provided with a slit, and the tip end of the lid sheet 12 may be inserted in the slit of the lid spool 40. In this manner, the base sheet 12 is wound around the base spool 40. In the present embodiment, the base spool 40 includes a mechanism for rolling up the base sheet 12. That is, the lid spool 40 is coupled with a lid spool gear (described below) for rotary drive. Hence, the lid spool 40 is rotated in a circumferential direction, centering on a rotation shaft extending in the direction perpendicular to the bottom plate 21. As a rotary driving mechanism for rotating the lid spool 40, a known one can be employed. For example, as the rotary driving mechanism, the ratchet mechanism, in which the lid spool 40 is rotated in one direction but is restricted to be rotated in an opposite direction thereof, may be employed. Also, the lid spool gear coupled with the lid spool 40 is in association the center gear (described below), for example, so that the lid spool 40 may be rotated as much as a predetermined amount along with turning of the center gear. For example, a turning range of the center gear is limited by a stopper (illustration thereof is omitted) or the like, which causes the rotating amount of the lid spool 40 at a time to be controlled. Also, this center gear may mesh not only with the lid spool gear coupled with the lid spool 40 but also with the aforementioned base spool gear coupled with the base spool 30 to rotate the lid spool gear and the base spool gear at the same time. In this manner, the lid spool 40 is rotated as much as to roll up the lid sheet 12, peel the lid sheet 12 from the base sheet 11, and open one blister 11a. Accordingly, the patient can open one blister 11 a with one action of turning the center gear and prepare an appropriate amount of medicine. Also, a gear (described below) for rotating the center gear may be attached to a cover lever (described below) for opening/closing the inhalation opening provided in a below-mentioned air piece 50. In this case, at the same time as the cover lever is opened to open the inhalation opening, the center gear turns, the lid spool 40 is rotated as much as the predetermined amount in association with this, and the lid sheet 12 is rolled up. Accordingly, the patient can easily prepare the medicine for inhalation only with one action of opening the lid member.

As illustrated in Figs. 2 and 3, the indexing wheel housing chamber 120 is provided with the indexing wheel 70. The indexing wheel 70 functions to support the medicinal holding sheet 10 when the blisters 11a of the medicinal holding sheet 10 are opened one by one. That is, the indexing wheel 70 is formed approximately in a cylindrical shape and is rotated in a circumferential direction, centering on a rotation shaft extending in the direction perpendicular to the bottom plate 21. On a circumferential side surface of the indexing wheel 70, an outer surface of the base sheet 11 in the medicinal holding sheet 10 abuts. As illustrated in the figures, the circumferential side surface of the indexing wheel 70 is provided with a plurality of pocket portions 81 at regular intervals along the circumferential direction of the indexing wheel 70. Each pocket portion 81 is an inwardly recessed part of the indexing wheel 70 and one blister 11a (refer to Fig. 1) of the base sheet 11 fits therein. Thus, the interval to form the blisters 11a in the base sheet 11 and the interval to form the pocket portions 71 in the indexing wheel 70 correspond to each other. The indexing wheel 70 may be coupled with a known gear and be driven to be rotated or may be rotated freely along with advance of the base sheet 11. Also, for example, the gear that drives the indexing wheel 70 may mesh with the aforementioned gear that drives the lid spool 40, and the indexing wheel 70 may be driven to be rotated in association with the lid spool 40. As the lid spool 40 and the indexing wheel 70 are rotated in association with each other, the medicinal holding sheet 10 advances in a state of abutting on the circumferential side surface of the indexing wheel 70 while the lid sheet 12 is rolled up in the lid spool 40. The base sheet 11 and the lid sheet 12 constituting the medicinal holding sheet 10 are separated at a separation point 60 on the circumferential side surface of the indexing wheel 70 and are guided in directions away from each other. Meanwhile, the phrase "the base sheet 11 and the lid sheet 12 are "separated on the circumferential side surface" of the indexing wheel 70" means that the base sheet 11 and the lid sheet 12 are separated when the medicinal holding sheet 10 is in a state of abutting on the circumferential side surface of the indexing wheel 70.

As illustrated in Figs. 2 and 3, the indexing wheel housing chamber 120 is defined by being surrounded by the external wall, the first internal wall 24, and the second internal wall 25 except the entrance and the exit of the sheets, and the air piece 50 is fitted in the external wall defining this indexing wheel housing chamber 120 to constitute a part of the external wall. The air piece 50 has the inhalation opening through which the patient inhales the medicine encapsulated in the medicinal holding sheet 10. The shape of the inhalation opening is not particularly limited as long as it is designed in consideration with dispersion characteristics of the powdered medicine. Multiple kinds of the air piece 50 can be prepared for powdered medicines having different dispersion characteristics. The air piece 50 has the inhalation opening at a position opposed to the blister 11a after the medicinal holding sheet 10 is separated into the base sheet 11 and the lid sheet 12 to cause the blister 11a to be opened. The indexing wheel 70 is rotated as much as a predetermined amount by a rotary driving mechanism in a state in which the blister 11a of the base sheet 11 is fitted in the pocket portion 81 and temporarily stops when the blister 11a reaches a position opposed to the inhalation opening of the air piece 50. In this manner, the medicine encapsulated in the blister 11a is prepared at a position at which the patient can inhale the medicine.

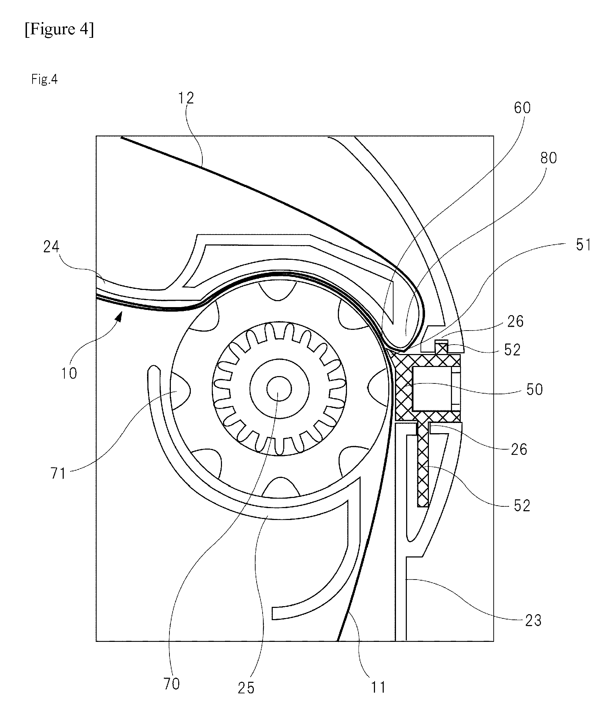

Fig. 4 is an enlarged view of a structure around the air piece 50 and the indexing wheel 70. As illustrated in Fig. 4, in the present invention, the air piece 50 having the inhalation opening has a wedge portion 51 to be inserted between the base sheet 11 and the lid sheet 12. Forming the wedge portion 51 in the air piece 50 in this manner is one of characteristics of the present invention. As illustrated in Fig. 4, the air piece 50 is provided with the wedge portion 51 at a position closest to the separation point 60 at which the medicinal holding sheet 10 is separated into the base sheet 11 and the lid sheet 12. The wedge portion 51 extends from a main body of the air piece 50 to a position at which a tip end thereof reaches the separation point 60. In the present embodiment, the wedge portion 51 is in a gradually tapered shape from the main body of the air piece 50 toward the separation point 60, and the tip end of the wedge portion 51 forms an acute angle. Locating the tip end of the wedge portion 51 at the separation point 60 of the base sheet 11 and the lid sheet 12 in this manner enables the base sheet 11 and the lid sheet 12 to be separated easily. Since the wedge portion 51 is inserted between the base sheet 11 and the lid sheet 12, the sheets are separated easily only by rotary-driving the lid spool 40 to roll up the lid sheet 12 without rotating the base spool 30 to roll up the base sheet 11, for example.

More specifically, the tip end of the wedge portion 51 of the air piece 50 forms the acute angle and is tapered. The angle of the tip end of the wedge portion 51 is preferably 10 to 80 degrees or 20 to 60 degrees, and more preferably 30 to 45 degrees, for example.

As illustrated in Fig. 4, the wedge portion 51 of the air piece 50 is arranged with the base sheet 11 of the medicinal holding sheet 10 interposed between the tip end thereof and a surface of the circumferential side surface of the indexing wheel 70. In other words, the base sheet 11 a of the medicinal holding sheet 10 passes between the tip end of the wedge portion 51 and the surface of the indexing wheel 70. Thus, in a case in which a thickness of an area in the base sheet 11a in which no blisters 11a are formed is T₁, a distance between the tip end of the wedge portion 51 and the surface of the indexing wheel 70 is preferably T₁ to 10T₁ or 2T₁ to 5T₁, for example. In this manner, the wedge portion 51 is inserted between the base sheet 11 and the lid sheet 12 in a state in which the base sheet 11 contacts the surface of the indexing wheel 70 to enable the sheets to be separated more easily.

Also, as illustrated in Figs. 2 to 4, the first internal wall 24 dividing the lid sheet housing chamber 110 from the indexing wheel housing chamber 120 is provided at an entrance part at which the lid sheet 12 is guided to the lid sheet housing chamber 110 with a guide portion 80 for guiding the lid sheet 12. That is, the entrance in the lid sheet housing chamber 110 is formed by a space between this guide portion 80 and the external wall. In the present embodiment, the guide portion 80 is integrally formed as a part of the first internal wall 24. When the lid sheet 12 is peeled from the base sheet 11 at the separation point 60 and advances in the opposite direction of the base sheet 12, an outer surface of the lid sheet 12 abuts on the guide portion 80. Apart of the guide portion 80 on which the lid sheet 12 abuts is rounded so that the outer surface of the lid sheet 12 may slide on the guide portion 80 smoothly. As illustrated in Fig. 4, in a preferred embodiment of the present invention, at least the tip end of the wedge portion 51 of the air piece 50 is located between the surface of the circumferential side surface of the indexing wheel 70 and the guide portion 80. Thus, at a stage at which the medicinal holding sheet 10 is in a state of abutting on the circumferential side surface of the indexing wheel 70 and is located in a space between the indexing wheel 70 and the guide portion 80, the wedge portion 51 is inserted between the base sheet 11 and the lid sheet 12, and the sheets are separated. Accordingly, the wedge portion 51 of the air piece 50 has the base sheet 11 interposed between the tip end thereof and the surface of the circumferential side surface of the indexing wheel 70 and has the lid sheet 12 interposed between the tip end thereof and the guide portion 80. In a case in which a thickness of the lid sheet 12 is T₂, a distance between the tip end of the wedge portion 51 and the guide portion 80 is preferably T₂ to 10T₂ or 2T₂ to 5T₂, for example. In this manner, the wedge portion 51 is inserted between the base sheet 11 and the lid sheet 12 in a state in which the lid sheet 12 contacts the guide portion 80 to enable the sheets to be separated more reliably.

As described above, in the preferred embodiment of the present invention, the tip end of the wedge portion 51 is located between the indexing wheel 70 and the guide portion 80 to form the separation point 60 of the base sheet 11 and the lid sheet 12. Thus, an angle θ of the mutually separated base sheet 11 and lid sheet 12 with the separation point 60 serving as a vertex is 1 or more and less than 90 degrees (1° ≤ θ < 90°). This angle θ is preferably 10 to 80 degrees or 20 to 60 degrees, and more preferably 30 to 45 degrees. In a conventional inhalation device, in general, the angle with the separation point serving as the vertex is 90 or more degrees in separating the lid sheet from the base sheet. In the case in which the angle with the separation point serving as the vertex is 90 or more degrees, both the base sheet 11 and the lid sheet 12 need to be pulled and rolled up, in which case relatively large tensile forces must be applied to both the sheets. In this respect, in the device according to the present invention, by employing a configuration in which the tip end of the wedge portion 51 is located between the indexing wheel 70 and the guide portion 80, the angle of the base sheet 11 and lid sheet 12 with the separation point 60 serving as the vertex can be less than 90 degrees, and this enables both the sheets to be separated easily.

Also, the wedge portion 51 of the air piece 50 is preferably made of a flexible material. The main body of the air piece 50 and the wedge portion 51 may be made of the flexible material integrally, or the main body of the air piece 50 and the wedge portion 51 may be made of different materials, and only the wedge portion 51 may be made of the flexible material. Examples of the flexible material to be used for the wedge portion 51 are polyacetal, polyamide, polycarbonate, a styrene-butadiene copolymer, flexible polyethylene, and flexible polypropylene. The wedge portion 51 is made of the flexible material to enable the base sheet 11 and the lid sheet 12 to be separated smoothly by the wedge portion 51.

Also, the wedge portion 51 of the air piece may contain an antistatic material. The main body of the air piece 50 and the wedge portion 51 may contain the antistatic material, or the main body of the air piece 50 and the wedge portion 51 may be made of different materials, and only the wedge portion 51 may contain the antistatic material. The antistatic material is one mixed into a material constituting the wedge portion 51 so that surface resistivity of the wedge portion 51 may be 10¹² Ω or less, for example, in order to efficiently eliminate static electricity to be generated by friction between the base sheet 11 and the wedge portion 51 and to prevent the powdered medicine from attaching to the wedge portion 51. Examples of such an antistatic material are a wetting material, surfactant (nonionic surfactant, anionic surfactant, and the like), and a conductive material.

Also, as illustrated in Figs. 2 to 4, the air piece 50 is formed separately from the external wall of the inhalation device. The external wall of the inhalation device is provided with cut-outs 26, and the air piece 50 is provided with projections 52 to be fitted in the cut-outs 26 of the external wall. The two cut-outs 26 of the external wall are provided at positions at which they are opposed with the air piece 50 interposed therebetween, and the two projections 52 of the air piece 50 are provided at positions corresponding to the cut-outs 26. By doing so, the air piece 50 is detachable from the external wall. In this manner, the air piece 50 is preferably replaceable. In the case in which the air piece 50 is replaceable, only the air piece 50 can be detached from the external wall and be cleaned, for example. Also, even in a case in which the air piece 50 is exhausted, the inhalation device 20 can be recycled by replacement of only the air piece 50. Further, for example, by preparing multiple kinds of air piece such as an air piece having a screw-shaped inhalation opening and air pieces having mesh structures with different aperture dimensions, the dispersion characteristics of the powdered medicine can be controlled in accordance with the patient. It is to be noted that a configuration making the air piece 50 replaceable is not limited to one described above. For example, the air piece 50 can be provided with a cut-out, and the external wall of the inhalation device 20 can be provided with a projection to be fitted in the cut-out of the air piece 50.

As in the above configuration, the medicinal holding sheet 10, which is in a state in which the base sheet 11 and the lid sheet 12 are attached to each other, is housed in the holding sheet housing space 90 in a rolled state. As for the medicinal holding sheet 10, the idle area provided at the tip end of the base sheet 11 and the lid sheet 12 is separated. The idle area at the tip end of the base sheet 11 is guided to the indexing wheel housing chamber 120, passes between the indexing wheel 70 and the wedge portion 51, is guided to the base sheet housing chamber 100, and is rolled up in the base spool 30. Also, the idle area at the tip end of the lid sheet 12 is guided to the indexing wheel housing chamber 120, passes between the guide portion 80 of the first internal wall 24 and the wedge portion 51, is guided to the lid sheet housing chamber 110, and is rolled up in the lid spool 40. In this manner, preparation for inhalation of the medicine with use of the inhalation device 20 is completed.

When the preparation for inhalation is completed, the patient performs an action of turning the base spool 30 and the lid spool 40, for example. For example, when the patient opens the cover lever (described below) covering the opening of the air piece 50, the base spool 30 and the lid spool 40 are rotated as much as predetermined amounts in predetermined directions via a gear mechanism. Through rotation of the base spool 30 and the lid spool 40, the base sheet 11 and the lid sheet 12 are rolled up, and the entire medicinal holding sheet 10 advances. Along with the advance of the medicinal holding sheet 10, the indexing wheel 70 is rotated. As a result, the blisters 11a of the base sheet 11 are fitted in the pocket portions 71 of the indexing wheel 70, and the base sheet 11 is fed to the base sheet housing chamber 110 along with rotation of the indexing wheel 70. The base sheet 11 and the lid sheet 12 of the medicinal holding sheet 10 advance in a state of abutting on the circumferential side surface of the indexing wheel 70, and the wedge portion 51 formed in the air piece 50 is inserted between the sheets 11 and 12. The wedge portion 51 is inserted to cause the separation point 60 of the base sheet 11 and the lid sheet 12 to be formed on the circumferential side surface of the indexing wheel 70. The separated lid sheet 12 passes between the wedge portion 51 and the guide portion 80, is guided by the guide portion 80, and is fed to the lid sheet housing chamber 110. Also, the separated base sheet 11 is guided by the indexing wheel 70, advances, and temporarily stops at the position at which the blister 11a is opposed to the inhalation opening of the air piece 50. By doing so, the patient can inhale the medicine encapsulated in the blister 11a of the base sheet 11 via the inhalation opening by holding the air piece 50 in his/her mouth and sucking. By repeating the above procedure, the blisters 11a of the medicinal holding sheet 10 can be opened one by one. As the procedure of opening the blisters 11a progresses, a roll diameter of the base sheet 11 rolled up in the base spool 30 and a roll diameter of the lid sheet 12 rolled up in the lid spool 40 are enlarged. That is, the state gradually changes from the state illustrated in Fig. 2 to the state illustrated in Fig. 3.

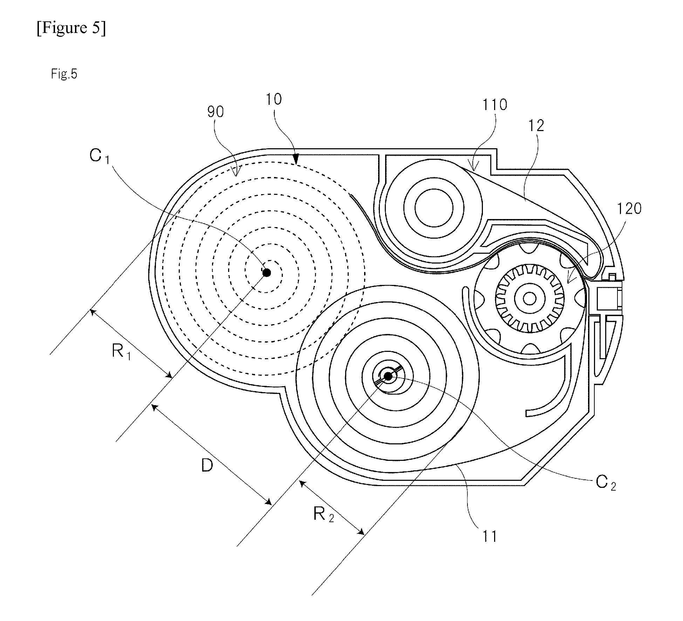

Fig. 5 illustrates comparison between a state in which the medicinal holding sheet 10 in the unused state is located in the holding sheet housing chamber 90 and a state in which the base sheet 11 in the used state is located in the base sheet housing chamber 100. In Fig. 5, the medicinal holding sheet 10 in the unused state is shown with the dashed line while the base sheet 11 in the used state is shown with the solid line. Here, "the unused state" means a state in which all of the blisters 11a are unopened while "the used state" means a state in which all of the blisters 11a are opened. As illustrated in Fig. 5, in the unused state, in which all of the blisters 11a are unopened, the rolled medicinal holding sheet 10 is partially located in the base sheet housing chamber 100. Conversely, in the used state, in which all of the blisters 11a are opened, the base sheet 11 wound around the base spool 30 is partially located in the holding sheet housing chamber 90. That is, as illustrated in Fig. 5, when the medicinal holding sheet 10 in the unused state and the base sheet 11 in the used state are compared, they partially overlap with each other. As the procedure of opening the blisters 11a progresses, a roll diameter of the medicinal holding sheet 10 housed in the holding sheet housing chamber 90 gradually decreases while the roll diameter of the base sheet 11 housed in the base sheet housing chamber 100 gradually increases. Thus, a distance between a center of the holding sheet housing chamber 90 and a center of the base sheet housing chamber 100 can be shortened to the extent that the housing chambers partially overlap with each other. By shortening the distance between the center of the holding sheet housing chamber 90 and the center of the base sheet housing chamber 100 in this manner, the entire inhalation device can be configured to be compact.

More specifically, as illustrated in Fig. 5, in a case in which the longest medicinal holding sheet 10 that can be housed in the holding sheet housing chamber 90 is wound in a rolled state, a radius of a largest circle diameter defined by the roll is referred to as R₁, and a center point of the largest circle diameter is referred to as C₁. Also, in a case in which the longest medicinal holding sheet 10 that can be housed in the holding sheet housing chamber 90 is used, and in which the base sheet 11 is guided to the base sheet housing chamber 100 and is wound in a rolled state, a radius of a largest circle diameter defined by the roll is referred to as R₂, and a center point of the largest circle diameter is referred to as C₂. At this time, a value for a linear distance D from the center point C₁ to the center point C₂ is smaller than a sum of the radius R₁ and the radius R₂ (D < R₁ + R₂). For example, in a case in which a value for R₁ + R₂ is 100%, the value for the linear distance D is preferably 10% to 95%, 15% to 70%, or 20% to 50%. By shortening the distance between the center of the holding sheet housing chamber 90 and the center of the base sheet housing chamber 100 in this manner, the entire inhalation device can be configured to be compact.

### (3. Roll-up Mechanism of Inhalation Device)

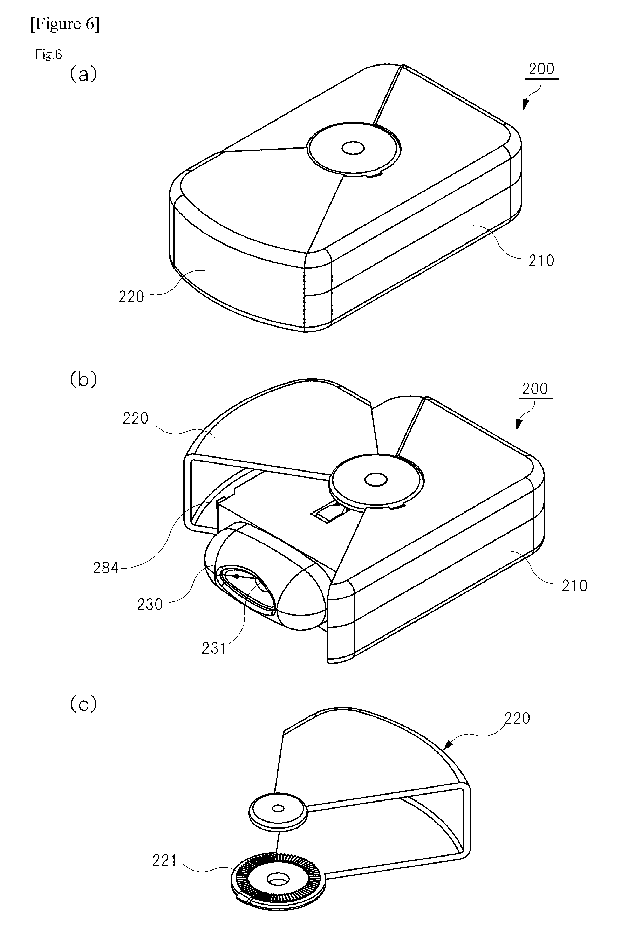

Next, referring to Figs. 6(a) to 9, a roll-up mechanism for the medicinal holding sheet included in the inhalation device will be described. It is to be noted that the inhalation device according to the present invention is not limited to one including a roll-up mechanism described below. In the inhalation device according to the present invention, the medicinal holding sheet can arbitrarily be rolled up by a known roll-up mechanism.

Fig. 6(a) is a perspective view illustrating appearance of an inhalation device 200. In Fig. 6(a), the inhalation device 200 is seen from a side of the bottom plate 21 illustrated in Figs. 2 and 3. As illustrated in Fig. 6(a), the inhalation device 200 includes an exterior case 210 and a cover lever 220. The exterior case 210 is a chassis housing the internal structure illustrated in Figs. 2 and 3. Also, the cover lever 220 is a lid member for opening/closing the inhalation opening for the medicine. The exterior case 210 and the cover lever 220 collectively constitute an outer shell of the inhalation device 200.

Fig. 6(b) is a perspective view illustrating the appearance in a state of opening the cover lever 220. As illustrated in Fig. 6(b), by opening the cover lever 220, a mouthpiece 230 is exposed. The mouthpiece 230 has an inhalation opening 231 for the medicine. The inhalation opening 231 of the mouthpiece 230 is linked with the opening of the air piece 50 illustrated in Figs. 2 and 3. Thus, the patient can inhale the medicine encapsulated in the medicinal holding sheet by sucking via the inhalation opening 231 of the air piece 50. The mouthpiece 230 is attached to the exterior case 210. The mouthpiece 230 is preferably detachable from the exterior case 210 so that the mouthpiece 230 can be cleaned.

Fig. 6(c) is a perspective view illustrating a state in which only the cover lever 220 is seen from a rear side. As illustrated in Fig. 6(c), a concave-convex lever gear 221 is formed on a rear side of a turning pivot point of the cover lever 220. This lever gear 221 meshes with a below-mentioned center gear. Thus, the cover lever 220 is opened with the lever gear 221 serving as the turning pivot point to cause the center gear to be rotated along with rotation of the cover lever 220.

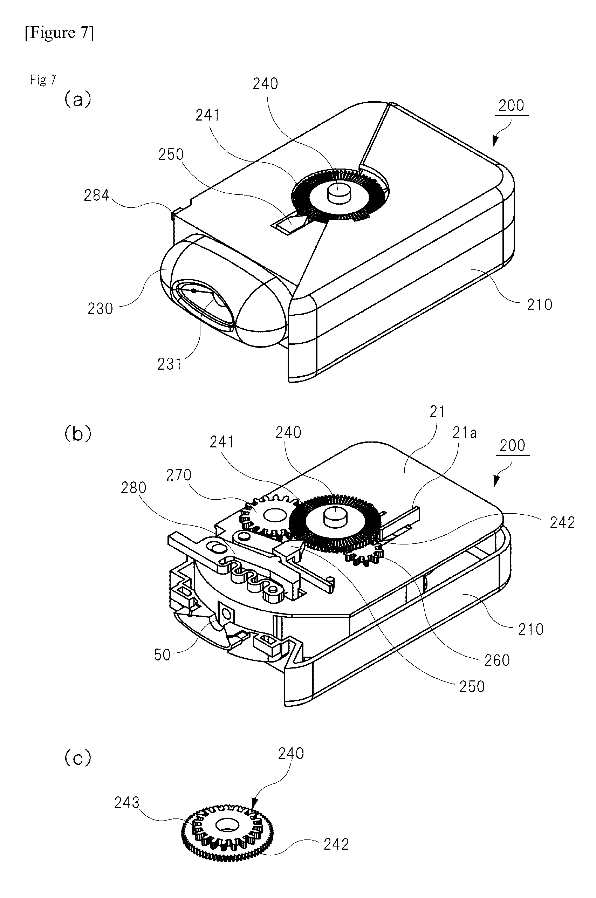

Fig. 7(a) is a perspective view illustrating a state in which the aforementioned cover lever 220 has been detached. As illustrated in Fig. 7(a), a center gear 240 is provided at a position facing the lever gear 221 of the cover lever 220 illustrated in Fig. 6. The center gear 240 has an outer convexity-and-concavity 241. As described above, the outer convexity-and-concavity 241 of the center gear 240 meshes with the lever gear 221 included in the cover lever 220. Thus, by opening the cover lever 220, a roll-up force thereof is transmitted to the center gear 240. Also, the outer convexity-and-concavity 241 formed on the center gear 240 is a so-called one-way clutch. That is, when the cover lever 220 is opened, the lever gear 221 and the outer convexity-and-concavity 241 of the center gear 240 mesh with each other, and the center gear 240 is rotated together with the lever gear 221. However, when the over lever 220 is closed, the lever gear 221 and the outer convexity-and-concavity 241 of the center gear 240 do not mesh with each other, and the center gear 240 is not rotated. Meanwhile, the exterior case 210 is provided with an opening at a part thereof at which the center gear 240 is located so that the lever gear 221 and the center gear 240 can mesh with each other.

Also, Fig. 7(a) illustrates a roll-up stop pawl 250 engaged with the center gear 240 to stop rotation of the center gear 240. The roll-up stop pawl 250 is engaged with a side convexity-and-concavity 242 provided on a circumferential side surface of the center gear 240 when the cover lever 220 is opened to stop rotation of the center gear 240. Thereafter, when the cover lever 220 is closed, the engagement of the roll-up stop pawl 250 with the center gear 240 is released. Providing the roll-up stop pawl 250 in this manner enables the patient to be prevented from rolling up the center gear 240 without closing the cover lever 220 once after he/she opens the cover lever 220 and inhales the medicine. This prevents the patient from inhaling the medicine consecutively by mistake.

Fig. 7(b) illustrates a state in which a rotary driving mechanism for rotating the base spool 30 and the lid spool 40 illustrated in Figs. 2 and 3 is exposed by detaching a bottom-side case of the exterior case 210. Also, Fig. 7(c) illustrates a state in which the center gear 240 illustrated in Fig. 7(b) is seen from a rear side. As illustrated in Fig. 7(b), the rotary driving mechanism includes the center gear 240, a base spool gear 260, and a lid spool gear 270. As illustrated in Fig. 7(c), the center gear 240 includes an inner convexity-and-concavity 243 on an opposite surface of the outer convexity-and-concavity 241 as well as the outer convexity-and-concavity 241 meshing with the lever gear 221 of the cover lever 220 and the side convexity-and-concavity 242 engaged with the roll-up stop pawl 250. The inner convexity-and-concavity 243 of the center gear 240 meshes with the base spool gear 260 and the lid spool gear 270. The base spool gear 260 is coupled with the base spool 30 illustrated in Figs. 2 and 3 and rotates the base spool 30. The lid spool gear 270 is coupled with the lid spool 40 illustrated in Figs. 2 and 3 and rotates the lid spool 40. Thus, a roll-up force generated by rotation of the center gear 240 is transmitted via the inner convexity-and-concavity 243 to the base spool gear 260 and the lid spool gear 270 and rotates the base spool 30 and the lid spool 40. Also, the bottom plate 21 arranged in the exterior case 210 is provided with a reverse rotation preventing pawl 21a engaged with the side convexity-and-concavity 242 of the center gear 240 to prevent reverse rotation of the center gear 240.

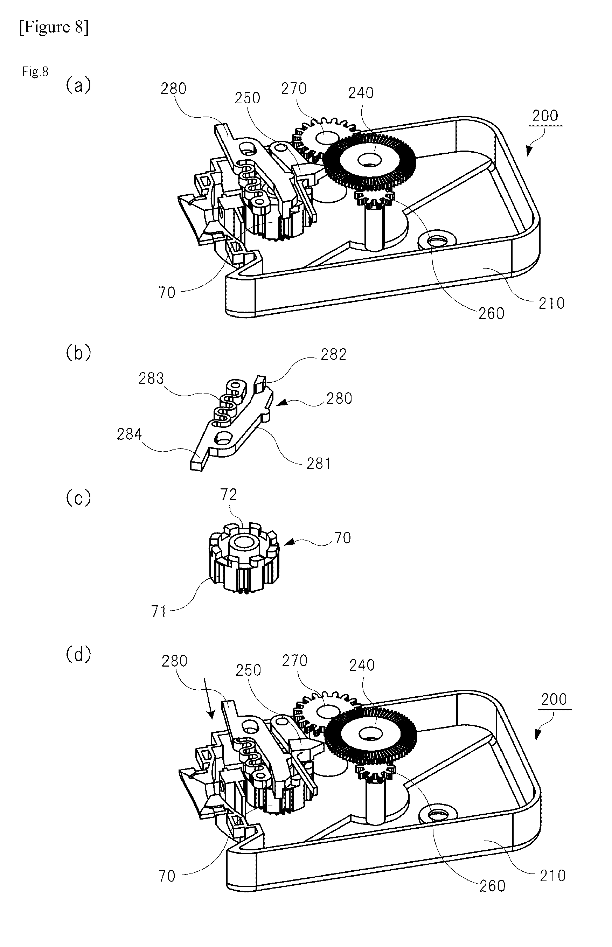

Also, the inhalation device includes a lock mechanism for stopping rotation of the center gear 240. The lock mechanism includes the roll-up stop pawl 250 engaged with the side convexity-and-concavity 242 of the center gear 240 and a rocker arm 280 biasing the roll-up stop pawl 250 in a direction of the center gear 240. Figs. 8(a) to 8(d) are perspective views illustrating a state in which the bottom plate 21 arranged in the exterior case 210 has been detached. Specifically, Fig. 8(a) illustrates a state in which the center gear 240 is locked by the roll-up stop pawl 250. Also, Fig. 8(b) illustrates a state in which the rocker arm 280 is seen from a rear side. Further, Fig. 8(c) illustrates the indexing wheel 70 solely extracted. Still further, Fig. 8(d) illustrates a state in which lock of the center gear 240 by the roll-up stop pawl 250 is released.

As illustrated in Fig. 8(a), the roll-up stop pawl 250 is located between the rocker arm 280 and the center gear 240. A pawl tip of the roll-up stop pawl 250 is engaged with the side convexity-and-concavity 242 of the center gear 240 in a state of being biased by the rocker arm 280. Accordingly, the roll-up stop pawl 250 can stop rotation of the center gear 240.

Also, as illustrated in Fig. 8(b), the rocker arm 280 includes a pressing portion 281, a projecting portion 282, a spring portion 283, and a lever portion 284 and is a member into which these elements are integrated. The pressing portion 281 abuts on the roll-up stop pawl 250 to apply a pressing force to the roll-up stop pawl 250. The projecting portion 282 is provided on a rear side of the pressing portion 281 and is fitted in a below-mentioned lock groove 72 of the indexing wheel 70. The spring portion 283 generates a biasing force for biasing the pressing portion 281 in a direction of the roll-up stop pawl 250. The lever portion 284 retracts the pressing portion 281 when the cover lever 220 is closed to release pressing toward the roll-up stop pawl 250.

Also, as illustrated in Fig. 8(c), the indexing wheel 70 is provided along a rotating direction thereof with the plurality of lock grooves 72 in which the projecting portion 282 of the rocker arm 280 is fitted, as well as the pocket portions 71 in which the blisters 11a of the base sheet 11 are fitted.

Here, operations of the lock mechanism will be described. First, when the patient opens the cover lever 220, the lock mechanism is in a locked state. As illustrated in Fig. 8(a), in the locked state, the projecting portion 282 of the rocker arm 280 is fitted in the lock groove 72 of the indexing wheel 70, and the pressing portion 281 presses the roll-up stop pawl 250, receiving the biasing force of the spring portion 283. The roll-up stop pawl 250 is pressed to cause the pawl tip to be engaged with the side convexity-and-concavity 242 of the center gear 240 so as to lock rotation of the center gear 240.

The patient inhales the medicine in a state in which the center gear 240 is locked. The patient thereafter closes the cover lever 220.

When the cover lever 220 is closed, the lever portion 284 of the rocker arm 280 is pressed in an arrow direction illustrated in Fig. 8(d) by the cover lever 220 (refer to Fig. 8). As illustrated in Fig. 8(d), the projecting portion 282 of the rocker arm 280 then goes out of the lock groove 72 of the indexing wheel 70, and the pressing portion 281 is retracted, to release pressing toward the roll-up stop pawl 250. Also, as the pressing portion 281 is retracted, the roll-up stop pawl 250 moves away from the side convexity-and-concavity 242 of the center gear 240. As a result, the center gear 240 is in a turnable state. The patient thereafter opens the cover lever 220 again to cause the center gear 240 to be rotated, which enables the medicinal holding sheet to be rolled up via a gear rotating mechanism.

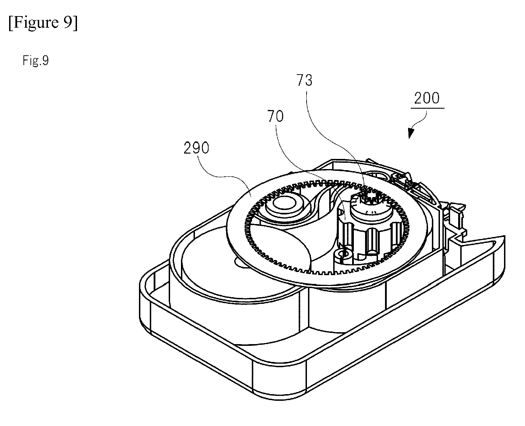

Fig. 9 illustrates an association mechanism of a counter dial in the inhalation device 200. Fig. 9 is a perspective view illustrating a state in which the internal structure of the inhalation device is seen from a side of the ceiling plate. As illustrated in Fig. 9, the inhalation device is equipped therein with a ring-like counter dial 290. "Numbers" or the like each representing the number of times of use are written on a surface of the counter 290 although illustration thereof is omitted. Also, convexity-and-concavity is formed on an inner circumferential side of the counter dial 290. On the other hand, as illustrated in Fig. 9, the indexing wheel 70 includes a counter gear 73 meshing with the convexity-and-concavity of the counter dial 290. Thus, the counter dial 290 is rotated in association with rotation of the indexing wheel 70. In this manner, since the counter dial 290 is rotated as much as a predetermined amount along with a predetermined amount of rotation of the indexing wheel 70, the numbers written on the counter 290 move. For example, by providing a ceiling surface side of the inhalation device with a window through which the patient looks into the number on the counter dial 290, the patient can look at the number on the counter dial 290 and check the number of times of use of the medicinal holding sheet and the number of remaining medicines.

In the present description, preferred embodiments of the present invention have been described above with reference to the drawings to express the content of the present invention. However, the present invention is not limited to the above embodiments and includes modified or improved embodiments apparent to those skilled in the art based on the matters described in the present description.

### Industrial Applicability

The present invention relates to a medicinal inhalation device. Accordingly, the present invention can be applied in a pharmaceutical industry, for example.

### Reference Signs List

10 ... medicinal holding sheet, 11 ... base sheet, 11a ... blister, 12 ... lid sheet 20 ... inhalation device, 21 ... bottom plate, 23 ... side plate, 24 ... first internal wall, 25 ... second internal wall, 26 ... cut-out, 30 ... base spool, 40 ... lid spool, 60 ... separation point, 50 ... air piece, 51 ... wedge portion, 52 ... projection, 70 ... indexing wheel, 71 ... pocket portion, 72 ... lock groove, 73 ... counter gear, 80 ... guide portion, 90 ...holding sheet housing chamber, 100 ... base sheet housing chamber, 110 ... lid sheet housing chamber, 120 ... indexing wheel housing chamber 200 ... inhalation device, 210 ... exterior case, 220 ... cover lever, 221 ... lever gear, 230 ... mouthpiece, 231 ... inhalation opening, 240 ... center gear, 241 ... outer convexity-and-concavity, 242 ... side convexity-and-concavity, 243 ... inner convexity-and-concavity, 250 ... roll-up stop pawl, 260 ... base spool gear, 270 ... lid spool gear, 280 ... rocker arm, 281 ... pressing portion, 282 ... projecting portion, 283 ... spring portion, 284 ... lever portion

## Claims

1. An inhalation device (20) housing a medicinal holding sheet (10) including an elongated base sheet (11) having a plurality of blisters (11a) encapsulating a medicine and an elongated lid sheet (12) attached to the base sheet (11),
wherein the inhalation device (20) enables to separate the lid sheet (12) from the base sheet (11),
wherein the inhalation device comprises:
an air piece (50) provided at a position opposed to the blister (11a) of the base sheet (11) from which the lid sheet (12) is separated and having an inhalation opening through which the medicine passes,
wherein the air piece (50) has a wedge portion (51) to be inserted between the base sheet (11) and the lid sheet (12) at a separation point (60) at which the lid sheet (12) is separated from the base sheet (11), and
the wedge portion (51) extends from a main body of the air piece (50) to a position at which a tip end thereof reaches the separation point (60).

2. The inhalation device according to claim 1, further comprising:
an indexing wheel (70) rotates in a state in which the medicinal holding sheet (10) abuts on a circumferential side surface of the indexing wheel,
wherein the wedge portion (51) of the air piece (50) is formed at a position at which the separation point (60) is formed on the circumferential side surface of the indexing wheel (70).

3. The inhalation device according to claim 2, further comprising:
a guide portion (80) on which an outer surface of the lid sheet (12) abuts when the lid sheet (12) is peeled from the base sheet (11) at the separation point (60) and advances in an opposite direction of the base sheet (12),
wherein the tip end of the wedge portion (51) is located between the indexing wheel (70) and the guide portion (80).

4. The inhalation device according to claim 1, wherein an angle of the mutually separated base sheet (11) and lid sheet (12) with the separation point (60) serving as a vertex is 1 or more and less than 90 degrees.
